# EUROPÄISCHE PATENTSCHRIFT

(11) **EP 2 872 233 B1**
(45) Veröffentlichungstag und Bekanntmachung des Hinweises auf die Patenterteilung: **14.09.2016**
(21) Anmeldenummer: 13734447.9
(22) Anmeldetag: 09.07.2013
(51) Int. Cl.: B01D 17/04, C07C 7/00

(54) **ABTRENNUNG VON IONISCHEN FLÜSSIGKEITEN MITTELS KOALESZIERFILTER AUS ACRYLPHENOLHARZ**
SEPARATION OF IONIC LIQUIDS USING AN ACRYLIC PHENYLIC RESIN COALESCING FILTER
ISOLEMENT DE LIQUIDES IONIQUES À L'AIDE D'UN FILTRE DE COALESCENCE EN RÉSINE ACRYLO- PHÉNOLIQUE

(30) Priorität: 11.07.2012 EP 12175856
(43) Veröffentlichungstag der Anmeldung: 20.05.2015
(73) Patentinhaber: BASF SE, 67056 Ludwigshafen am Rhein (DE)
(72) Erfinder: PFEIFFER, Daniel, 67435 Neustadt (DE); BITTERLICH, Stefan, 67246 Dirmstein (DE)
(86) Internationale Anmeldenummer: PCT/EP2013/064459
(87) Internationale Veröffentlichungsnummer: WO 2014/009353

(56) Entgegenhaltungen:
- DE-A1-102005 029 423
- US-A1- 2007 017 875
- US-A1- 2010 130 800

## Beschreibung

Die vorliegende Erfindung betrifft ein Verfahren zur Abtrennung einer Phase (A), die mindestens eine ionische Flüssigkeit enthält, von einer Phase (B) in einem Koaleszierfilter (K), der aus Acrylphenolharz hergestellt ist, wobei die Phase (A) eine höhere Viskosität als die Phase (B) aufweist.

Ionische Flüssigkeiten eignen sich unter anderem als Katalysatoren für die Isomerisierung von Kohlenwasserstoffen. Eine entsprechende Verwendung einer ionischen Flüssigkeit ist beispielsweise in WO 2011/069929 offenbart, wo eine spezielle Auswahl von ionischen Flüssigkeiten in Gegenwart eines Olefins zur Isomerisierung von gesättigten Kohlenwasserstoffen eingesetzt wird, insbesondere zur Isomerisierung von Methylcyclopentan (MCP) zu Cyclohexan.

Im Allgemeinen sind ionische Flüssigkeiten einerseits und Kohlenwasserstoffe (bzw. organische Phasen generell) andererseits nicht oder nur sehr schwer mischbar, sie bilden zwei getrennte Phasen aus. Um die genannte Katalysewirkung nutzen zu können, muss ein intensiver Kontakt zwischen organischer Phase und der ionischer Flüssigkeit hergestellt werden. Hierzu werden die beiden Phasen häufig in Rührkesseln und intensivem Rühren unter Erhalt von Dispersionen durchmischt. In Abhängigkeit von Parametern wie Art der ionischen Flüssigkeit bzw. der organischen Phase oder dem Phasenverhältnis kann die Dispersion entweder als Dispersion einer ionischen Flüssigkeit in der organischen Phase vorliegen oder es kann sich um eine Dispersion der organischen Phase in der ionischen Flüssigkeit handeln. Unabhängig von der konkret vorliegenden Dispergierrichtung ist es bei solchen Dispersionen ein generelles Problem, die dispergierte Phase im Anschluss an die Reaktion von der zusammenhängenden Phase abzutrennen. Problematisch ist dabei insbesondere die Fallkonstellation, bei der Feinsttropfen der ionischen (d< 900 µm) Flüssigkeit aus einer Dispersion abgetrennt werden sollen, in der die ionische Flüssigkeit in der organischen Phase dispergiert ist (Feinsttropfenproblem).

Zur Auftrennung von zwei- oder mehrphasigen Gemischen, insbesondere von Dispersionen, ist die Verwendung von Koaleszierfiltern seit längerem bekannt. Beispielsweise wird in der internationalen Anmeldung PCT/IB2012/050417 (angemeldet am 30.01.2012) ein Verfahren zur Reduzierung des Wassergehaltes in Pyrolysebenzin unter Verwendung eines Koaleszierfilters, der aus Metall und/oder Glasfaser hergestellt ist, offenbart. Ein Koaleszierfilter kann jedoch nicht nur zur Wasserabtrennung aus Gemischen (Dispersionen) mit einer organischen Phase (Pyrolysebenzin) verwendet werden, sondern auch zur Abtrennung von ionischen Flüssigkeiten aus Dispersionen mit einer organischen Phase.

WO 2010/062922 offenbart ein mehrstufiges Verfahren zur Trennung einer ionischen Flüssigkeit von Kohlenwasserstoffen unter Verwendung eines Koaleszierfilters. Das Koaleszierfiltermaterial muss so beschaffen sein, dass es eine stärkere Affinität für die ionische Flüssigkeit als für die Kohlenwasserstoffe hat. Als Koaleszierfiltermaterialien eignen sich gemäß WO 2010/062922 Glaskugeln, Edelstahl, Glasfaser, Polymerfasern oder organische Membrane, insbesondere Glasfasern. Im Koaleszierfilter wird eine Trennung der ionischen Flüssigkeit aus den Kohlenwasserstoffen bewirkt.

Die der vorliegenden Erfindung zugrunde liegende Aufgabe besteht in der Bereitstellung eines neuen Verfahrens zur Abtrennung einer ionischen Flüssigkeit aus einer organischen Phase, wobei die ionische Flüssigkeit in der organischen Phase dispergiert ist.

Gelöst wird die Aufgabe durch ein Verfahren zur Abtrennung einer Phase (A), die mindestens eine ionische Flüssigkeit enthält, von einer Phase (B), wobei die Phase (A) eine höhere Viskosität als die Phase (B) aufweist, umfassend folgende Schritte:
a) Bereitstellung eines Stroms (S1) enthaltend eine Dispersion (D1), in der die Phase (A) in der Phase (B) dispergiert ist,
b) Einleiten des Stroms (S1) in einen Koaleszierfilter (K), der aus Acrylphenolharz hergestellt ist,
c) Abtrennen der dispergierten Phase (A) von der Phase (B) im Koaleszierfilter (K),
d) Ausleiten eines Stroms (S2) umfassend mindestens 70 Gew.-%, bevorzugt mindestens 90 Gew-.% an Phase (A) aus dem Koaleszierfilter (K), und
e) Ausleiten eines Stroms (S3) umfassend mindestens 70 Gew.-%, bevorzugt mindestens 90 Gew-% an Phase (B) aus dem Koaleszierfilter (K).

Die eingesetzten Koaleszierfilter aus Acrylphenolharz zeichnen sich zunächst dadurch aus, dass sie (unter den für das vorliegende Verfahren relevanten Rahmenbedingungen) im Gegensatz zu anderen Koaleszierfiltern (beispielsweise Polyphenylensulfid) stabiler sind und/oder über einen längeren Zeitraum ihre (Trenn-) Leistung (Koaleszierwirkung) aufrechterhalten.

Durch das erfindungsgemäße Verfahren kann in vorteilhafter Weise eine effektive Abtrennung von ionischen Flüssigkeiten aus Dispersionen mit organischen Phasen, insbesondere aus Dispersionen mit Kohlenwasserstoffen, erzielt werde. Erfindungsgemäß kann insbesondere das Problem der Abtrennung von in feindisperser Form und/oder in kleinen Mengen in einer Dispersion mit einer organischen Phase vorliegenden ionischen Flüssigkeit gelöst werden (Feinsttropfenproblem).

Es können durch das erfindungsgemäße Verfahren auch kleinere Mengen (< 1 Gew.-%) an ionischer Flüssigkeit aus einer Dispersion mit einer organischen Phase, insbesondere mit einer Kohlenwasserstoffphase, abgetrennt werde; dies gilt insbesondere auch für den Fall, dass eine Dispergierrichtung von ionischer Flüssigkeit in organischer Phase vorliegt. Durch gezielte Einstellung der Dispergierrichtung von Phase (A; ionische Flüssigkeit) in Phase (B; organische Phase) kann eine große (schnelle) Trenngeschwindigkeit erzielt werden, mit der Konsequenz dass die Größe der im Verfahren verwendeten Phasenscheider deutlich reduziert werden kann gegenüber dem Fall der umgekehrten Dispergierrichtung - Phase (B; organische Phase) in Phase (A; ionische Flüssigkeit).

Das erfindungsgemäße Verfahren kann also unabhängig davon durchgeführt werden, welche Dispergierrichtung in den vorrausgegangen Verfahrensschritten vorliegt. Liegt beispielsweise in einem vorausgegangenen Isomerisierungsschritt eine Dispergierrichtung mit Phase (B) in Phase (A) vor, weil beispielsweise bei der Isomerisierung ein deutlicher Überschuss an ionischer Flüssigkeit eingesetzt wird, kann in einer bevorzugten Ausführungsform der vorliegenden Erfindung problemlos ein Kippen der Dispergierrichtung durchgeführt werden. Das Kippen der Dispergierrichtung wird erfindungsgemäß durchgeführt, indem ein Strom, der einen Überschuss an organischer Phase enthält, vor den Koaleszierfilter aus Acrylphenolharz und insbesondere auch vor einen dem Koaleszierfilter vorgeschalteten zusätzlichen Phasenscheider rückgeleitet wird.

Nachfolgend wird das erfindungsgemäße Verfahren zur Abtrennung von ionischen Flüssigkeiten mittels Koaleszierfiltern aus Acrylphenolharz näher definiert.

Die Phase (A) enthält mindestens eine ionische Flüssigkeit. Beispielsweise können in der Phase (A) Gemische aus zwei oder mehr ionischen Flüssigkeiten enthalten sein, vorzugsweise enthält die Phase (A) eine ionische Flüssigkeit. Neben der ionischen Flüssigkeit können in der Phase (A) auch weitere Komponenten enthalten sein, die mit der ionischen Flüssigkeit mischbar sind. Bei solchen Komponenten kann es sich beispielsweise um Cokatalysatoren handeln, die bei Isomerisierungsreaktionen unter Verwendung von ionischen Flüssigkeiten eingesetzt werden. Bevorzugtes Beispiel für solche Cokatalysatoren sind Halogenwasserstoffe, insbesondere Chlorwasserstoff. Darüber hinaus können in der Phase (A) auch Bestandteile oder Zerfallsprodukte der ionischen Flüssigkeiten, die beispielsweise bei der durch die ionische Flüssigkeit katalysierte Reaktion entstehen können, enthalten sein, wie Aluminiumchlorid. Vorzugsweise ist bei der Phase (A) der Anteil an ionischer Flüssigkeit größer 80 Gew.-% (bezogen auf die Summe aller Komponenten der Phase (A)).

Als ionische Flüssigkeiten eignen sich im Rahmen der vorliegenden Erfindung prinzipiell alle dem Fachmann bekannten ionischen Flüssigkeiten, sofern sie die durchzuführende Reaktion wie z.B. Isomerisierung katalysieren.. Ein Überblick hinsichtlich zur Katalyse von Isomerisierungsreaktionen geeigneter ionischer Flüssigkeiten kann beispielsweise WO 2011/069929 entnommen werden. Bevorzugt ist im Rahmen der vorliegenden Erfindung eine saure ionische Flüssigkeit. Vorzugsweise ist die in der Phase (A) enthaltene ionische Flüssigkeit eine saure ionische Flüssigkeit mit der Zusammensetzung K1AlₙX₍₃ₙ₊₁₎, wobei K1 ein einwertiges Kation, X gleich Halogen und 1 < n < 2,5, ist. K1 ist vorzugsweise ein unsubstituiertes oder zumindest teilweise alkyliertes Ammoniumion oder ein heterocyclisches (einwertiges) Kation, insbesondere ein Pyridiniumion, ein Imidazoliumion, ein Pyridaziniumion, ein Pyrazoliumion, ein Imidazoliniumion, ein Thiazoliumion, ein Triazoliumion, ein Pyrrolidiniumion, ein Imidazolidiniumion oder ein Phosphoniumion. X ist vorzugsweise Chlor oder Brom.

Mehr bevorzugt enthält die saure ionische Flüssigkeit als Kation ein zumindest teilweise alkyliertes Ammoniumion oder ein heterocyclisches Kation und/oder als Anion ein Chloroalumination mit der Zusammensetzung AlₙCl₍₃ₙ₊₁₎ mit 1 < n < 2,5. Vorzugsweise enthält das zumindest teilweise alkylierte Ammoniumion einen, zwei oder drei Alkylreste mit (jeweils) 1 bis 10 Kohlenstoffatomen. Sofern zwei oder drei Alkylsubstituenten mit den entsprechenden Ammoniumionen vorhanden sind, kann die jeweilige Kettenlänge unabhängig voneinander gewählt werden, vorzugsweise weisen alle Alkylsubstituenten die gleiche Kettenlänge auf. Besonders bevorzugt sind trialkylierte Ammoniumionen mit einer Kettenlänge von 1 bis 3 Kohlenstoffatomen. Das heterocyclische Kation ist vorzugsweise ein Imidazoliumion oder ein Pyridiniumion.

Besonders bevorzugt enthält die saure ionische Flüssigkeit als Kation ein zumindest teilweise alkyliertes Ammoniumion und als Anion ein Chloroalumination mit der Zusammensetzung AlₙCl₍₃ₙ₊₁₎ mit 1 < n < 2,5. Beispiele für solche besonders bevorzugten ionischen Flüssigkeiten sind Trimethylammoniumchloroaluminat und Triethylammoniumchloroaluminat.

Im Rahmen der vorliegenden Erfindung weist die Phase (A) eine höhere Viskosität als die Phase (B) auf. Vorzugsweise weist die Phase (A) eine um mindestens 0,1 mPas, insbesondere um mindestens 20 mPas, höhere Viskosität aufweist als die Phase (B).

Die Phase (B) ist im Rahmen der vorliegenden Erfindung zunächst dadurch charakterisiert, dass sie gegenüber der Phase (A) eine niedrigere Viskosität aufweist. Beispielsweise kann es sich bei der Phase (B) um eine organische Phase handeln. Vorzugsweise enthält die Phase (B) mindestens einen Kohlenwasserstoff. Mehr bevorzugt enthält die Phase (B) als Kohlenwasserstoff Cyclohexan oder ein Gemisch aus Cyclohexan mit mindestens einem weiteren Kohlenwasserstoff, ausgewählt aus Methylcyclopentan (MCP), n-Hexan, iso-Hexan, n-Heptan, iso-Heptan oder Dimethylcyclopentan. Besonders bevorzugt enthält die Phase (B) ein Gemisch aus Cyclohexan, MCP und mindestens einem weiteren Kohlenwasserstoff.

Im Rahmen der vorliegenden Erfindung erfolgt in Schritt a) die Bereitstellung eines Stroms (S1) enthaltend eine Dispersion (D1), in der die Phase (A) in der Phase (B) dispergiert ist. Die Dispergierrichtung (d.h. die Information, welche Phase in disperser Form in der der jeweils anderen Phase vorliegt) kann bestimmt werden, indem eine Probe, ggf. nach Zusatz eines selektiv die eine Phase einfärbenden Farbstoffs, unter einem Lichtmikroskop mit Durchlicht untersucht wird.

Die Dispersion (D1) kann nach dem Fachmann bekannten Methoden hergestellt werden, beispielsweise kann eine solche Dispersion durch intensives Verrühren der in den jeweiligen Phasen enthaltenen Komponenten erzeugt werden. Ein solcher Vorgang kann beispielsweise im Rahmen eines Isomerisierungsverfahrens von Kohlenwasserstoff unter Verwendung einer ionischen Flüssigkeit stattfinden. Die Dispersion (D1) wird (wie nachfolgend noch näher ausgeführt) bevorzugt als Oberphase aus einer Phasentrennvorrichtung entnommen, die besonders bevorzugt einer Vorrichtung nachgeschaltet ist, in der eine durch die ionische Flüssigkeit katalysierte Reaktion durchgeführt wird und in der die ionische Flüssigkeit und die organische Phase unter Rührung in Kontakt gebracht werden. In der Dispersion (D1) können die Phasen (A) und (B) in beliebigen Verhältnissen zueinander vorliegen, unter der Voraussetzung, dass die Phase (A) in der Phase (B) dispergiert ist. Vorzugsweise ist im Strom (S1) in der Dispersion (D1) die Phase (A) zu maximal 10 Gew.-%, insbesondere zu maximal 5 Gew.-% enthalten (jeweils bezogen auf die Menge an Phase (B)).

Gemäß dem erfindungsgemäßen Schritt b) erfolgt das Einleiten des Stroms (S1) in einen Koaleszierfilter (K), der aus Acrylphenolharz hergestellt ist. Geeignete Koaleszierfilter (K) sind dem Fachmann bekannt, sie sind beispielsweise kommerziell erhältlich von der Firma Fuhr GmbH (Deutschland) oder von dem Hersteller CUNO Fluid Purification. Solche geeigneten Koaleszierfilter (K) haben Feinheiten von 1-150 µm, bevorzugt 10, 25 oder 50 µm, besonders bevorzugt 10 µm. Weiterhin sind zwei Versionen bezüglich der Oberfläche möglich: gerillt und ungerillt, bevorzugt ist ungerillt. Die Kerzen des Koaleszierfilter (K) als solche haben beispielsweise einen Innendurchmesser von 27 mm und einen Außendurchmesser von 65 mm und sind in Längen von 4" bis 60" erhältlich. Die Kerze ist vorzugsweise eine asymmetrische, harzgebundene und stützkernlose Filterkerze. Vorzugsweise enthält sie im Wesentlichen Acrylfasern, die mit Phenolharz gebunden werden.

Wie beispielsweise aus dem Ausführungsbeispiel der vorliegenden Erfindung ersichtlich, kann der Koaleszierfilter (K) in eine größere Einheit, beispielsweise einen Filterbehälter, integriert sein. Vorzugsweise wird im Rahmen der vorliegenden Erfindung unter einem Koaleszierfilter (K), der aus Acrylphenolharz hergestellt ist, das Filtermaterial als solches verstanden. Die sonstigen Komponenten der Filtereinheit, beispielsweise der Behälter der Einheit (Filterbehälter) oder das Filtermodul, in dem das Filtermaterial eingebracht ist, können aus anderen Materialien als Acrylphenolharz hergestellt sein. Der Begriff "hergestellt aus" bedeutet im Rahmen der vorliegenden Erfindung, dass das zur Herstellung des Filtermaterials verwendete Material Acrylphenolharz enthält. Vorzugsweise enthält das Filtermaterial mindestens 50 Gew.-%, mehr bevorzugt mindestens 75 Gew.-% und insbesondere mindestens 95 Gew.-% Acrylphenolharz.

In Schritt c) erfolgt das Abtrennen der dispergierten Phase (A) von der Phase (B) im Koaleszierfilter (K). Die Durchführung des Abtrennens als solche - wobei aufgrund der Koaleszierwirkung des Koaleszierfilters (K) voneinander abgetrennte Phasen (A) und (B) erhalten werden - mittels eines Koaleszierfilters (K) ist dem Fachmann bekannt.

Gemäß Schritt d) erfolgt im erfindungsgemäßen Verfahren das Ausleiten eines Stroms (S2) umfassend mindestens 70 Gew.-%, bevorzugt mindestens 90 Gew.-% an Phase (A) aus dem Koaleszierfilter (K). Besonders bevorzugt ist im Strom (S2) keine oder nur eine geringe Menge an Phase (B) enthalten (< 1 Gew.-%). Die vorstehenden Angaben in Gew.-% beziehen sich auf die entsprechenden Mengenangaben, die im Strom (S1) enthalten sind.

In Schritt e) erfolgt das Ausleiten eines Stroms (S3) umfassend mindestens 70 Gew.-%, bevorzugt mindestens 90 Gew-% an Phase (B) aus dem Koaleszierfilter (K). Besonders bevorzugt ist im Strom (S2) keine oder nur eine geringe Menge an Phase (A) enthalten (< 1 Gew.-%). Die vorstehenden Angaben in Gew.-% beziehen sich auf die entsprechenden Mengenangaben, die im Strom (S1) enthalten sind.

Vorzugsweise wird der in Schritt a) bereitgestellte Strom (S1) aus einer dem Koaelszierfilter (K) vorgeschalteten Phasentrenneinheit erhalten. Bei dieser Phasentrenneinheit handelt es sich vorzugsweise um einen Phasenscheider. Weiterhin ist es bevorzugt, dass der Phasentrenneinheit ein Reaktionsapparat oder eine Kaskade von Reaktionsapparaten vorgeschaltet ist. Bei diesem Reaktionsapparat oder der Kaskade von Reaktionsapparaten handelt es sich vorzugsweise um Vorrichtungen, die dazu geeignet sind, eine Isomerisierung von Kohlenwasserstoffen in Gegenwart von mindestens einer ionischen Flüssigkeit als Katalysator durchzuführen.

In einer bevorzugten Ausführungsform der vorliegenden Erfindung werden zusätzlich zu den vorstehend beschriebenen Schritten a) bis e) die nachfolgenden zusätzlichen Schritte f) bis k) durchgeführt, die wie folgt definiert sind:
f) Ausleitung eines Stroms (S4) aus dem Reaktionsapparat oder der Kaskade von Reaktionsapparaten, wobei (S4) eine Dispersion (D2) enthält, in der die Phase (B) in der Phase (A) dispergiert ist,
g) Einleiten eines Stroms (S5), enthaltend zu mindestens 70 Gew.-%, bevorzugt zu mindestens 90 Gew.-%, die Phase (B), in den Strom (S4), wobei der Strom (S5) aus Schritt k) rückgeführt wird, vorzugsweise werden die Ströme (S4) und (S5) mittels eines Rückorgans oder eines statischen Mischers vermischt,
h) unter Ausbildung eines Stroms (S6), enthaltend eine Dispersion (D1), in der die Phase (A) in der Phase (B) dispergiert ist,
i) Einleiten des Stroms (S6) in die Phasentrenneinheit, die dem Koaleszierfilter (K) vorgeschaltet ist,
j) Auftrennen des Stroms (S6) in der Phasentrenneinheit in einen Strom (S1), gemäß Schritt a) und in einen Strom (S7) enthaltend zu mindestens 70 Gew.-%, bevorzugt mindestens 90 Gew.-%, die Phase (A),
k) Abtrennung einer Teilmenge des Stroms (S1) und/oder einer Teilmenge des Stroms (S3) gemäß Schritt e) als Strom (S5) und Rückführung des Stroms (S5) nach Schritt g).

Im Rahmen der vorliegenden Erfindung kann der Strom (S5), der in Schritt g) in den Strom (S4) eingeführt wird, aus einer Teilmenge des Stromes (S1) gebildet werden. Alternativ dazu kann der Strom (S5) auch aus einer Teilmenge des Stromes (S3) gebildet werden. Gegebenenfalls kann der Strom (S5) auch aus unterschiedlichen oder gleichen Teilmengen der Ströme (S1) und (S3) gebildet werden. Vorzugsweise wird der Strom (S5) aus einer Teilmenge des Stromes (S1) gebildet. Beispielsweise werden zwischen 50 und 90 % der Ströme (S1) und/oder (S3) als Strom (S5) abgetrennt und in den Strom (S4) rückgeführt. Allerdings ist es auch denkbar, dass zumindest temporär größere Mengen oder die entsprechenden Ströme sogar vollständig rückgeleitet werden. Durch die Rückleitung von Teilmengen der Ströme (S1) und/oder (S3) als Strom (S5) und der damit verbundenen Einleitung des Stromes (S5) in den Strom (S4) wird ein Kippen der Dispergierrichtung im Strom (S4) erzielt. Kippen der Dispergierrichtung bedeutet, dass im Strom (S4) eine Dispersion (D2) enthalten ist, bei der die Phase (B) in der Phase (A) dispergiert ist und dass durch geeignete Wahl der Menge des Stromes (S5) in Schritt g) ein Strom (S6) ausgebildet, wird, der die Dispersion (D1) enthält, in der die Phase (A) in der Phase (B) dispergiert ist. Sofern vor dem Koaleszierfilter (K) eine Phasentrenneinheit, insbesondere ein Phasenscheider vorgeschaltet ist, wird in der Dispersion (D1) der Anteil an Phase (A) Weiter reduziert, was sich vorteilhaft auf die Trennleistung im Koaleszierfilter (K) auswirkt.

Vorzugsweise erfolgt in Schritt g) das Einleiten des Stroms (S5) in den Strom (S4) in einer Rühr- oder Mischvorrichtung, in der der Strom (S6) gemäß Schritt h) ausgebildet wird.

Weiterhin ist es bevorzugt, dass das Phasenverhältnis der Phase (A) zur Phase (B) in der im Strom (S6) enthaltenen Dispersion (D1) ≤ 3 [kg/kg], bevorzugt ≤ 0,9 [kg/kg], ist.

Weiterhin ist es bevorzugt, dass der Strom (S4) aus einer Isomerisierung in Gegenwart einer ionischen Flüssigkeit, insbesondere einer Isomerisierung von Methylcyclopentan (MCP) zu Cyclohexan in Gegenwart einer ionischen Flüssigkeit, erhalten wird.

Weiterhin ist es bevorzugt, dass in Schritt k) die Abtrennung von Strom (S5) aus Strom (S1) außerhalb der Phasentrenneinheit erfolgt.

Gegebenenfalls können der aus der Phasentrenneinheit gemäß Schritt j) abgetrennte Strom (S7) und/oder der aus dem Koaleszierfilter (k) ausgeleitete Strom (S2) gemäß Schritt d), die jeweils die Phase (A) enthalten, in den Reaktionsapparat oder die Kaskade von Reaktionsapparaten rückgeleitet werden. Gegebenenfalls können der Strom (S7) und/oder der Strom (S2) auch an eine andere Stelle des erfindungsgemäßen Verfahrens rückgeleitet werden, beispielswiese in eine Misch-oder Rührvorrichtung, um die Konzentration der Phase (A) in der Dispersion (D1) zu steuern.

Aus dem Strom (S3) wird im Rahmen der vorliegenden Erfindung vorzugsweise Cyclohexan isoliert. Verfahren und Vorrichtungen zur Abtrennung von Cyclohexan aus dem Strom (S3), insbesondere wenn es sich um ein Kohlenwasserstoffgemisch handelt, sind dem Fachmann bekannt. Gegebenenfalls können vor der Abtrennung des Cyclohexans noch weitere Aufreinigungsschritte (beispielsweise eine Wäsche mit einer wässrigen und/oder alkalischen Phase) durchgeführt werden, die dem Fachmann bekannt sind.

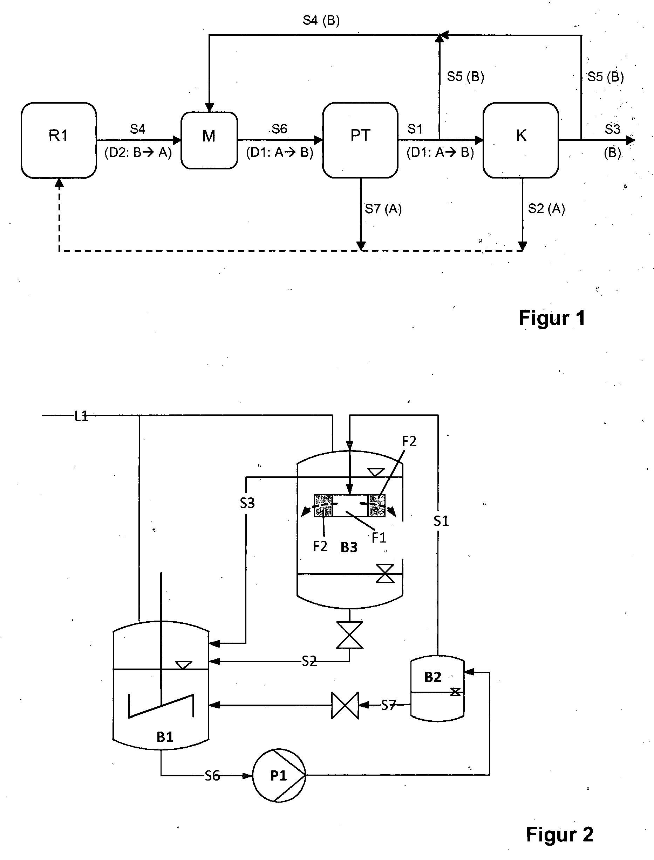

In Figur 1 wird das erfindungsgemäße Verfahren (einer Ausgestaltung) der vorstehend beschriebenen bevorzugten Ausführungsform nochmals verdeutlicht. Gemäß Figur 1 wird das Verfahren so durchgeführt, dass sowohl aus dem Strom (S1) als auch aus dem Strom (S3) eine Teilmenge als Strom (S5) in den Strom (S4) rückgeführt wird. Zum besseren Verständnis sind in Figur 1 unter den jeweiligen Strömen in Klammern die darin enthaltenen Hauptkomponenten angegeben. Bei den Strömen (S1), (S4) und (S6) ist im jeweiligen Klammerausdruck auch die Dispergierrichtung der jeweiligen Dispersionen mit berücksichtigt, wobei der Pfeil die Dispergierrichtung zum Ausdruck bringt. Dies bedeutet, dass beispielsweise die im Strom (S4) enthaltene Dispersion (D2) eine Phase (B) aufweist, die in der Phase (A) dispergiert ist. In Figur 1 erfolgt die Einleitung des Stromes (S5) in den Strom (S4) in einer Mischvorrichtung (M). Durch die gestrichelte Linie ist angedeutet, dass die Ströme (S7) und/oder (S2) gegebenenfalls auch in den Reaktionsapparat oder eine Kaskade von Reaktionsapparaten (R1) rückgeleitet werden können. PT in Figur 1 bedeutet Phasentrenneinheit und K bedeutet Koaleszierfilter.

Nachfolgend wird die Erfindung anhand der Beispiele verdeutlicht.

### Beispiele

Für den Versuch werden die folgenden Substanzen verwendet:
Phase (A):
   Ionische Flüssigkeit (IL) mit der Zusammensetzung (CH₃)₃NH AlₙCl₃ₙ₊₁ mit n=1,82 laut Elementaranalyse (auch als IL-Phase bezeichnet).
Phase (B):
   Kohlenwasserstoff-Gemisch mit der Zusammensetzung (auch als organische Phase bezeichnet)
      - Methylcyclopentan 20 Gew.-%
      - Cyclohexan 50 Gew.-%
      - Hexan 28 %
      - Isohexane (technische Mischung) 2 Gew.-%

Für die beschriebenen Versuche werden die Phasen (A) und (B) im Mengenverhältnis 0,1 kg(A)/kg(B) eingesetzt.

### 1. Vergleich organischer Filtermaterialien hinsichtlich Beständigkeit

Für diesen Versuch werden zwei Filtermaterialien jeweils nach Entfernen des aus Edelstahl gefertigten Stützgitters in ein gerührtes Zweiphasengemisch aus den oben genannten Substanzen (A) und (B) eingetaucht und dort 20 h lang bei Raumtemperatur belassen.

Die Ergebnisse des Lagerversuchs sind wie folgt:

| **Filtermaterial** | **Aussehen nach Lagerung in IL** |
|---|---|
| PALL Profile RLS1FPS100 10µm (Polyphenylensulfid) | stellenweise starke Verfärbung (grüne Flecken) |
| Acrylphenolharz (Fa. Fuhr GmbH) 10µm, ungerillt | Unverändert - |

### 2. Filtrationstest mit dem Acrylphenolharzfilter

Die Versuchsanordnung ist in der Abbildung gemäß Figur 2 dargestellt:
In Behälter (B1) (2,5 I- Rührgefäß mit Glas-Scheibenrührer, mit 1000 U/min gerührt) wird ein Zweiphasengemisch aus (A) und (B) vorgelegt, wobei (A) in (B) dispergiert ist. B1 ist (wie auch B3) mit einer Stickstoff führenden Gasleitung (L1) verbunden, die auf Atmosphärendruck gehalten wird.

Mittels der Dosierpumpe (P1) wird die Dispersion (D1) als Strom (S6) in einen Phasenscheider (B2) geführt. Hier findet eine Grobtrennung zwischen den Phasen (A) und (B) statt. Die Phase (A) wird über Strom 7 nach B1 zurückgeführt, wobei das Ventil in Strom 7 so eingestellt wird, dass die Phasengrenze zwischen (A) und (B) während der Versuchsdauer ungefähr auf konstantem Niveau verharrt. Der bis auf eine geringe Menge Fremdphase (siehe unten) aus Phase (B) bestehende Strom (S1) wird über den Innenraum des Filtermoduls (F1) und das Filtermaterial (F2) in den Filterbehälter (B3) geführt. Das Filtermodul enthält das Acrylphenolharz-Filtermaterial gemäß Beschreibung unter Versuch 1 als Ring mit rechteckigem Querschnitt (Höhe 3 cm, Durchmesser 5 cm), das von einem aus Edelstahl gefertigten Stützgitter umgeben ist und zusammen mit diesem mittels einer Schraubvorrichtung zwischen zwei profilierten Platten aus Teflon gehaltert wird. Der Strom (S1) strömt durch eine Zuführungsleitung in den durch das Filtermaterial und die Halterplatten gebildeten Hohlraum und von dort radial nach außen in den Filterbehälter. Die dort anfallenden beiden flüssigen Phasen werden über einen Überlauf (Strom (S3), organische Phase) bzw. ein handgeregeltes Ventil (Strom (S2), IL-Phase) in B1 zurückgeführt. Die Druckdifferenz zwischen Innen- und Außenraum des Filtermoduls wird gemessen.

Alle Behälter sind mit einem Doppelmantel ausgestattet und werden während der nachfolgend beschriebenen Versuche mittels eines über einen Laborthermostaten zirkulierten Wärmeträgeröls auf 50 °C gehalten.

Zunächst wird Strom (S3) für 1 h auf 5 l/h eingestellt. Aus dem Filter treten dabei Tropfen der schweren Phase (an (A) reiche Phase) mit einem Durchmesser von 1 bis 2 mm aus. Der Druckverlust über das Filtermodul liegt unter 500 mbar. Das Ventil in Strom (S2) bleibt in dieser Zeit geschlossen, so dass sich im Unterteil von B3 ca. 250 ml schwere Phase ansammelt, die Phasengrenze liegt am Ende dieser Periode noch unter dem Filtermodul.

Danach wird die Pumpe angehalten und das Filtermodul wird für 10 Tage in der organischen Phase belassen. Danach wird B1 entleert und erneut mit der oben beschriebenen Dispersion (D1) aus (A) und (B) befüllt.

Danach wird P1 wieder in Betrieb genommen. Es werden zwei verschiedene Feedmengen zum Filter (Strom (S1)) eingestellt. Dabei wird das Ventil in Strom (S2) jeweils so eingestellt, dass die Höhe der Phasengrenze in B3 konstant bleibt. Nach 20 min werden vom Feedstrom zum Filter (Strom (S1)) Proben genommen und auf ihren Stickstoffgehalt analysiert. Die Größe der aus dem Filter austretenden Tropfen der schweren Phase wird visuell abgeschätzt.

Die Ergebnisse sind in der folgenden Tabelle dargestellt:

| Menge Strom (S1)[l/h] | Druckverlust Filter [mbar] | Größe Tropfen Phase (A) nach Filter [mm] | N-Gehalt [Gew.-ppm] | |
|---|---|---|---|---|
| | | | Strom (S1) | org. Phase |
| 4,8 | 0 | 2 - 3 | 65 | 7 |
| 8,1 | 0 | 3 | 65 | 17 |

Die Ergebnisse zeigen, dass der getestete Acrylphenolharzfilter für die Dauer des Versuchs stabil und auch wirksam ist (der N-Gehalt in der organischen Phase in B2 bzw. in Strom (S3) ist ein Maß für die Verunreinigung organischen Phase mit IL-Fremdphase nach dem Filter).

## Patentansprüche

1. Verfahren zur Abtrennung einer Phase (A), die mindestens eine ionische Flüssigkeit enthält, von einer Phase (B), wobei die Phase (A) eine höhere Viskosität als die Phase (B) aufweist, umfassend folgende Schritte:
a) Bereitstellung eines Stroms (S1) enthaltend eine Dispersion (D1), in der die Phase (A) in der Phase (B) dispergiert ist,
b) Einleiten des Stroms (S1) in einen Koaleszierfilter (K), der aus Acrylphenolharz hergestellt ist,
c) Abtrennen der dispergierten Phase (A) von der Phase (B) im Koaleszierfilter (K),
d) Ausleiten eines Stroms (S2) umfassend mindestens 70 Gew.-%, bevorzugt mindestens 90 Gew-.% an Phase (A) aus dem Koaleszierfilter (K), und
e) Ausleiten eines Stroms (S3) umfassend mindestens 70 Gew.-%, bevorzugt mindestens 90 Gew-% an Phase (B) aus dem Koaleszierfilter (K).

2. Verfahren gemäß Anspruch 1, **dadurch gekennzeichnet, dass** die Phase (A) eine um mindestens 0,1 mPas, insbesondere um mindestens 20 mPas, höhere Viskosität aufweist als die Phase (B).

3. Verfahren gemäß Anspruch 1 oder 2, **dadurch gekennzeichnet, dass** die Phase (B) mindestens einen Kohlenwasserstoff enthält.

4. Verfahren gemäß Anspruch 3, **dadurch gekennzeichnet, dass** die Phase (B) als Kohlenwasserstoff Cyclohexan oder ein Gemisch aus Cyclohexan mit mindestens einem weiteren Kohlenwasserstoff, ausgewählt aus Methylcyclopentan (MCP), n-Hexan, iso-Hexan, n-Heptan, iso-Heptan oder Dimethylcyclopentan, enthält.

5. Verfahren gemäß einem der Ansprüche 1 bis 4, **dadurch gekennzeichnet, dass** die in der Phase (A) enthaltene ionische Flüssigkeit eine saure ionische Flüssigkeit mit der Zusammensetzung K1AlₙX₍₃ₙ₊₁₎ ist, wobei K1 ein einwertiges Kation, X gleich Halogen und 1 < n < 2,5 ist.

6. Verfahren gemäß Anspruch 5, **dadurch gekennzeichnet, dass** die saure ionische Flüssigkeit als Kation ein zumindest teilweise alkyliertes Ammoniumion oder ein heterocyclisches Kation und/oder als Anion ein Chloroalumination mit der Zusammensetzung AlₙCl₍₃ₙ₊₁₎ mit 1 < n < 2,5 enthält.

7. Verfahren gemäß einem der Ansprüche 1 bis 5, **dadurch gekennzeichnet, dass** im Strom (S1) in der Dispersion (D1) die Phase (A) zu maximal 5 Gew.-% vorliegt.

8. Verfahren gemäß einem der Ansprüche 1 bis 7, **dadurch gekennzeichnet, dass** der Strom (S1) aus einer dem Koaleszierfilter vorgeschalteten Phasentrenneinheit erhalten wird, der wiederum ein Reaktionsapparat oder eine Kaskade von Reaktionsapparaten vorgeschaltet ist.

9. Verfahren gemäß Anspruch 8, **dadurch gekennzeichnet, dass** die Phasentrenneinheit ein Phasenscheider ist.

10. Verfahren gemäß Anspruch 8 oder 9 umfassend die folgenden zusätzlichen Schritte:
f) Ausleitung eines Stroms (S4) aus dem Reaktionsapparat oder der Kaskade von Reaktionsapparaten gemäß Anspruch 8, wobei (S4) eine Dispersion (D2) enthält, in der die Phase (B) in der Phase (A) dispergiert ist,
g) Einleiten eines Stroms (S5), enthaltend zu mindestens 70 Gew-.%, bevorzugt zu mindestens 90 Gew-.%, die Phase (B), in den Strom (S4), wobei der Strom (S5) aus Schritt k) rückgeführt wird,
h) unter Ausbildung eines Stroms (S6), enthaltend eine Dispersion (D1), in der die Phase (A) in der Phase (B) dispergiert ist,
i) Einleiten des Stroms (S6) in die Phasentrenneinheit, die dem Koaleszierfilter (K) vorgeschaltet ist,
j) Auftrennen des Stroms (S6) in der Phasentrenneinheit in einen Strom (S1), gemäß Schritt a) von Anspruch 1, und in einen Strom (S7) enthaltend zu mindestens 70 Gew.-%, bevorzugt mindestens 90 Gew.-%, die Phase (A),
k) Abtrennung einer Teilmenge des Stroms (S1) und/oder einer Teilmenge des Stroms (S3) gemäß Schritt e) von Anspruch 1 als Strom (S5) und Rückführung des Stroms (S5) nach Schritt g).

11. Verfahren gemäß Anspruch 10, **dadurch gekennzeichnet, dass** in Schritt g) das Einleiten des Stroms (S5) in den Strom (S4) in einer Rühr- oder Mischvorrichtung erfolgt, in der der Strom (S6) gemäß Schritt h) ausgebildet wird.

12. Verfahren gemäß Anspruch 10 oder 11, **dadurch gekennzeichnet, dass** das Phasenverhältnis der Phase (A) zur Phase (B) in der im Strom (S6) enthaltenen Dispersion (D1 ) ≤ 3 [kg/kg], bevorzugt ≤ 0,9 [kg/kg] ist.

13. Verfahren gemäß einem der Ansprüche 10 bis 12, **dadurch gekennzeichnet, dass** der Strom (S4) aus einer Isomerisierung erhalten wird, vorzugsweise einer Isomerisierung in Gegenwart einer ionischen Flüssigkeit, insbesondere einer Isomerisierung von Methylcyclopentan (MCP) zu Cyclohexan in Gegenwart einer ionischen Flüssigkeit.

14. Verfahren nach einem der Ansprüche 10 bis 13, **dadurch gekennzeichnet, dass** in Schritt k) die Abtrennung von Strom (S5) aus Strom (S1) außerhalb der Phasentrenneinheit erfolgt.

15. Verfahren gemäß einem der Ansprüche 1 bis 14, **dadurch gekennzeichnet, dass** aus dem Strom (S3) Cyclohexan isoliert wird.

## Claims

1. A process for separating a phase (A) comprising at least one ionic liquid from a phase (B), phase (A) having a higher viscosity than phase (B), comprising the following steps:
a) providing a stream (S1) comprising a dispersion (D1) in which phase (A) is dispersed in phase (B),
b) introducing stream (S1) into a coalescing filter (K) manufactured from acrylic/phenolic resin,
c) separating the dispersed phase (A) from phase (B) in the coalescing filter (K),
d) discharging a stream (S2) comprising at least 70% by weight, preferably at least 90% by weight, of phase (A) from the coalescing filter (K), and
e) discharging a stream (S3) comprising at least 70% by weight, preferably at least 90% by weight, of phase (B) from the coalescing filter (K).

2. The process according to claim 1, wherein the viscosity of phase (A) is at least 0.1 mPas and especially at least 20 mPas higher than that of phase (B).

3. The process according to claim 1 or 2, wherein phase (B) comprises at least one hydrocarbon.

4. The process according to claim 3, wherein phase (B) comprises, as the hydrocarbon, cyclohexane or a mixture of cyclohexane with at least one further hydrocarbon selected from methylcyclopentane (MCP), n-hexane, isohexane, n-heptane, isoheptane or dimethylcyclopentane.

5. The process according to any of claims 1 to 4, wherein the ionic liquid present in phase (A) is an acidic ionic liquid having the composition K1AlₙX₍₃ₙ₊₁₎ where K1 is a monovalent cation, X is halogen and 1 < n < 2.5.

6. The process according to claim 5, wherein the acidic ionic liquid comprises, as a cation, an at least partly alkylated ammonium ion or a heterocyclic cation and/or, as an anion, a chloroaluminate ion having the composition AlₙCl₍₃ₙ₊₁₎ where 1 < n < 2.5.

7. The process according to any of claims 1 to 5, wherein phase (A) is present to a maximum extent of 5% by weight in stream (S1) in dispersion (D1).

8. The process according to any of claims 1 to 7, wherein stream (S1) is obtained from a phase separation unit which is connected upstream of the coalescing filter and which is in turn connected downstream of a reaction apparatus or a cascade or reaction apparatuses.

9. The process according to claim 8, wherein the phase separation unit is a phase separator.

10. The process according to claim 8 or 9 comprising the following additional steps:
f) discharging a stream (S4) from the reaction apparatus or the cascade of reaction apparatuses according to claim 8, (S4) comprising a dispersion (D2) in which phase (B) is dispersed in phase (A),
g) introducing a stream (S5) comprising at least 70% by weight, preferably at least 90% by weight, of phase (B), into stream (S4), stream (S5) being recycled from step k),
h) to form a stream (S6) comprising a dispersion (D1) in which phase (A) is dispersed in phase (B),
i) introducing stream (S6) into the phase separation unit connected upstream of the coalescing filter (K),
j) separating stream (S6) in the phase separation unit into a stream (S1) according to step a) of claim 1, and into a stream (S7) comprising at least 70% by weight, preferably at least 90% by weight, of phase (A),
k) removing a portion of stream (S1) and/or a portion of stream (S3) according to step e) of claim 1 as stream (S5) and recycling stream (S5) to step g).

11. The process according to claim 10, wherein, in step g), stream (S5) is introduced into stream (S4) in a stirring or mixing apparatus in which stream (S6) according to step h) is formed.

12. The process according to claim 10 or 11, wherein the phase ratio of phase (A) to phase (B) in dispersion (D1) present in stream (S6) is ≤ 3 [kg/kg], preferably ≤ 0.9 [kg/kg].

13. The process according to any of claims 10 to 12, wherein stream (S4) is obtained from an isomerization, preferably an isomerization in the presence of an ionic liquid, especially an isomerization of methylcyclopentane (MCP) to cyclohexane in the presence of an ionic liquid.

14. The process according to any of claims 10 to 13, wherein, in step k), stream (S5) is removed from stream (S1) outside the phase separation unit.

15. The process according to any of claims 1 to 14, wherein cyclohexane is isolated from stream (S3).

## Revendications

1. Procédé de séparation d'une phase (A), qui contient au moins un liquide ionique, d'une phase (B), la phase (A) présentant une viscosité plus élevée que la phase (B), comprenant les étapes suivantes :
a) la préparation d'un courant (S1) contenant une dispersion (D1), dans laquelle la phase (A) est dispersée dans la phase (B),
b) l'introduction du courant (S1) dans un filtre à coalescence (K), qui est fabriqué en une résine d'acrylphénol,
c) la séparation de la phase dispersée (A) de la phase (B) dans le filtre à coalescence (K),
d) le déchargement d'un courant (S2) comprenant au moins 70 % en poids, de préférence au moins 90 % en poids, de la phase (A) du filtre à coalescence (K), et
e) le déchargement d'un courant (S3) comprenant au moins 70 % en poids, de préférence au moins 90 % en poids, de la phase (B) du filtre à coalescence (K).

2. Procédé selon la revendication 1, **caractérisé en ce que** la phase (A) présente une viscosité au moins 0,1 mPas, notamment au moins 20 mPas, plus élevée que la phase (B).

3. Procédé selon la revendication 1 ou 2, **caractérisé en ce que** la phase (B) contient au moins un hydrocarbure.

4. Procédé selon la revendication 3, **caractérisé en ce que** la phase (B) contient en tant qu'hydrocarbure du cyclohexane ou un mélange de cyclohexane avec au moins un autre hydrocarbure, choisi parmi le méthylcyclopentane (MCP), le n-hexane, l'iso-hexane, le n-heptane, l'iso-heptane ou le diméthylcyclopentane.

5. Procédé selon l'une quelconque des revendications 1 à 4, **caractérisé en ce que** le liquide ionique contenu dans la phase (A) est un liquide ionique acide de composition K1AlₙX₍₃ₙ₊₁₎, K1 étant un cation monovalent, X étant un halogène, et 1 < n < 2,5.

6. Procédé selon la revendication 5, **caractérisé en ce que** le liquide ionique acide contient en tant que cation un ion ammonium au moins partiellement alkylé ou un cation hétérocyclique, et/ou en tant qu'anion un ion chloroaluminate de composition AlₙCl₍₃ₙ₊₁₎, avec 1 < n < 2,5.

7. Procédé selon l'une quelconque des revendications 1 à 5, **caractérisé en ce que** la phase (A) est présente à hauteur d'au plus 5 % en poids dans la dispersion (D1) dans le courant (S1).

8. Procédé selon l'une quelconque des revendications 1 à 7, **caractérisé en ce que** le courant (S1) est obtenu à partir d'une unité de séparation de phases connectée en amont du filtre à coalescence, en amont de laquelle un appareil de réaction ou une cascade d'appareils de réaction est à son tour connecté.

9. Procédé selon la revendication 8, **caractérisé en ce que** l'unité de séparation de phases est un séparateur de phases.

10. Procédé selon la revendication 8 ou 9, comprenant les étapes supplémentaires suivantes :
f) le déchargement d'un courant (S4) de l'appareil de réaction ou de la cascade d'appareils de réaction selon la revendication 8, (S4) contenant une dispersion (D2) dans laquelle la phase (B) est dispersée dans la phase (A),
g) l'introduction d'un courant (S5), contenant au moins 70 % en poids, de préférence au moins 90 % en poids, de la phase (B), dans le courant (S4), le courant (S5) étant recyclé à partir de l'étape k),
h) pour former un courant (S6), contenant une dispersion (D1) dans laquelle la phase (A) est dispersée dans la phase (B),
i) l'introduction du courant (S6) dans l'unité de séparation de phases qui est connectée en amont du filtre à coalescence (K),
j) la séparation du courant (S6) dans l'unité de séparation de phases en un courant (S1), selon l'étape a) de la revendication 1, et en un courant (S7) contenant au moins 70 % en poids, de préférence au moins 90 % en poids, de la phase (A),
k) la séparation d'une partie du courant (S1) et/ou d'une partie du courant (S3) selon l'étape e) de la revendication 1 en tant que courant (S5) et le recyclage du courant (S5) selon l'étape g).

11. Procédé selon la revendication 10, **caractérisé en ce qu'**à l'étape g), l'introduction du courant (S5) dans le courant (S4) a lieu dans un dispositif d'agitation ou de mélange, dans lequel le courant (S6) selon l'étape h) est formé.

12. Procédé selon la revendication 10 ou 11, **caractérisé en ce que** le rapport entre la phase (A) et la phase (B) dans la dispersion (D1) contenue dans le courant (S6) est ≤ 3 [kg/kg], de préférence ≤ 0,9 [kg/kg].

13. Procédé selon l'une quelconque des revendications 10 à 12, **caractérisé en ce que** le courant (S4) est obtenu à partir d'une isomérisation, de préférence d'une isomérisation en présence d'un liquide ionique, notamment d'une isomérisation de méthylcyclopentane (MCP) en cyclohexane en présence d'un liquide ionique.

14. Procédé selon l'une quelconque des revendications 10 à 13, **caractérisé en ce qu'**à l'étape k), la séparation du courant (S5) du courant (S1) a lieu à l'extérieur de l'unité de séparation de phases.

15. Procédé selon l'une quelconque des revendications 1 à 14, **caractérisé en ce que** du cyclohexane est isolé à partir du courant (S3).
